# EUROPÄISCHE PATENTANMELDUNG

(11) **EP 0 665 202 A2**
(43) Veröffentlichungstag der Anmeldung: **02.08.1995**
(21) Anmeldenummer: 95105012.9
(22) Anmeldetag: 10.02.1992
(51) Int. Cl.: C07C 17/087, C07C 19/01

(54) **Verfahren zur Herstellung von 2,2-Dichlorpropan**

(30) Priorität: 22.02.1991 DE 4105532; 27.09.1991 DE 4132211
(62) Teilanmeldung aus: 92102155.6
(71) Anmelder: BAYER AG, 51368 Leverkusen (DE)
(72) Erfinder: Arlt, Dieter, Prof. Dr., D-51065 Köln (DE)

(57) **Zusammenfassung**

2-Chlorpropen, das im Gemisch mit 1-Chlorpropenen vorliegt, läßt sich mit Chlorwasserstoff selektiv zum 2,2-Dichlorpropan umsetzen, das sich von den nicht umgesetzten 1-Chlorpropenen leicht abtrennen läßt.

## Beschreibung

Die Erfindung betrifft ein Verfahren zur Herstellung von 2,2-Dichlorpropan aus Gemischen von 2-Chlorpropen und 1-Chlorpropenen.

Es wurde gefunden, daß es möglich ist, durch selektive Umsetzung von 2-Chlorpropen, das im Gemisch mit 1-Chlorpropenen vorliegt, mit Chlorwasserstoff ein Gemisch aus 2,2-Dichlor-propan und 1-Chlorpropenen zu erhalten, das z.B. durch fraktionierende Destillation sehr leicht in 1-Chlorpropen-Gemisch und reines 2,2-Dichlorpropan aufgetrennt werden kann. Dadurch wird die aufwendige Trennung von 2- und 1 -Chlorpropenen umgangen (2-Chlorpropen: Siedepunkt 22,6°C, E/Z-1-Chlorpropene: Siedebereich 30,8°C bis 37°C, 2,2-Dichlorpropan: Siedepunkt 69,5°C). Es ist bekannt, daß sowohl 2- als auch E- und Z-1-Chiorpropen bei der Umsetzung mit Chlorwasserstoff HCl-Additionsprodukte ergehen, aber es ist überraschend, daß die Addition an 2-Chlorpropen soviel schneller als an 1-Chlor-propene erfolgt, so daß sich daraus ein vorteilhaftes Herstellungsverfahren für reines 2,2-Dichlorpropan ergibt. Die Umsetzung kann unter Normaldruck oder Überdruck durchgeführt werden; vorteilhaft wird im Bereich von 2 bis 50 bar gearbeitet.

Die Verwendung von 2-Chlorpropen im Gemisch mit 1-Chlor-propenen, wobei das Gemisch vorzugsweise unter 80 Gew.-% 1-Chlorpropene (bezogen auf die Summe von 1- und 2-Chlorpropenen) enthält - ist vorteilhaft, weil dieser Ausgangsstoff leicht aus 1,2-Dichlorpropan durch HCl-Abspaltung zugänglich ist, wobei Gemische von 1- und 2-Chlor-propenen anfallen, deren aufwendige Trennung bei der vorgeschlagenen Verwendung entfallen kam.

Da 1,2-Dichlorpropan Zwangsanfall-Produktbei der technischen Herstellung von Propenoxid ist, stellt die vorgeschlagene Verwendung von 2-Chlor-propen eine ökologisch besonders vorteilhafte Verfahrensweise zur Verfügung, da sie ein mit erheblichem Aufwand zu entsorgendes Abfallprodukt nutzbar macht.

Die Umsetzung kann unkatalysiert ausgeführt werden; zweckmäßig ist es jedoch, die HCl-Addition durch Zugabe geeigneter Katalysatoren zu beschleunigen. Katalysatoren des erfindungsgemäßen Verfahren umfassen Friedel-Crafts-Katalysatoren, die überlicherweise für die Alkylierung von aromatischen Verbindungen verwendeten Friedel-Crafts-Katalysatoren eingesetzt werden, z.B. AlCl₃, BF₃, FeCl₃, BiCl₃, ZnCl₂, TiCl₄, ZrCl₄, SnCl₄, H₃PO₄, HF, HBF₄, saure Erdalkaliphosphate, saure Tonerden, Zeolithe oder saure Ionenaustauscherharze.

Geeignet sind weiterhin Katalysatoren, die zwar die HCl-Addition an ungesättigte Verbindungen katalysieren, jedoch nicht als Friedel-Crafts-Katalysatoren bei der Alkylierung von aromatischen Kohlenwasserstoffen wirksam sind, wie z.B. Kupferhalogenide, Quecksilbersalze oder Bariumchlorid, wobei diese Stoffe auch in Form heterogener Katalysatoren auf Trägern, wie z.B. Aktivkohle, angewendet werden können.

Die Reaktionstemperaturen können -78 bis +90°C betragen.

Der gasförmige Chlorwasserstoff, der mit 2-Chlorpropen umgesetzt wird, wird in mindestens äquivalenter Menge eingesetzt, kann aber auch im Überschuß, z.B. 2 bis 10 Mol pro Mol 2-Chlorpropen, eingesetzt werden.

Der Reaktionsablauf wird zweckmäßig durch gaschromatographische Untersuchung des Reaktionsgemisches verfolgt.

Die Prozentangaben des nachfolgenden Beispiels beziehen sich auf das Gewicht.

### Beispiel

275 g eines Chlorpropengemisches, nach GC-Analyse 68,6 % E/Z-1-Chlorpropene und 27,8 % 2-Chlor-propen enthaltend, wurde unter Zusatz von 1,3 g wasserfreiem Eisen-(III)-chlorid in einem 1 l-Rührautoklaven bei 10 bar mit Chlorwasserstoffgas behandelt. Die Reaktionstemperatur betrug 50°C, die Reaktionszeit 3 Stunden. Nach der Umsetzung enthielt das Reaktionsgemisch nach GC-Analyse 58,0 % E/Z-1-Chlorpropene und 39,3 % 2,2-Dichlorpropan, d.h. die HCl-Addition erfolgte selektiv an das 2-Chlor-propen. Nach Behandlung des Rohgemisches mit Wasser und Trocknung der organischen Phase mit Calciumchlorid wurde das Gemisch durch fraktionierende Destillation an einer 60 cm-Füllkörperkolonne getrennt. Man erhielt 99 g (= 88 % d.Th.) 2,2-Dichlorpropan vom Siedebereich 68-70°C.

## Patentansprüche

1. Verfahren zur Herstellung von 2,2-Dichlorpropan aus Gemischen von 2-Chlor-propen und 1-Chlorpropenen durch selektive Umsetzung des 2-Chlorpropens mit Chlorwasserstoff und Abtrennung des resultierenden 2,2-Dichlorpropans aus dem Reaktionsgemisch.
